# FASCICULE DE BREVET EUROPEEN

(11) **EP 2 751 259 B1**
(45) Date de publication et mention de la délivrance du brevet: **14.11.2018**
(21) Numéro de dépôt: 12755992.0
(22) Date de dépôt: 03.09.2012
(51) Int. Cl.: C12N 5/0775, C12N 5/077, C12N 5/0735, C12N 5/00, C12N 13/00

(54) **PROCEDES D'AGREGATION ET DE DIFFERENCIATION DE CELLULES SOUCHES MAGNETISEES**
VERFAHREN ZUR AGGREGATION UND DIFFERENZIERUNG MAGNETISIERTER STAMMZELLEN
METHODS FOR AGGREGATION AND DIFFERENTIATION OF MAGNETIZED STEM CELLS

(30) Priorité: 01.09.2011 FR 1157755
(43) Date de publication de la demande: 09.07.2014
(73) Titulaire: Centre National de la Recherche Scientifique, 75016 Paris (FR)
(72) Inventeur: FAYOL, Delphine, F-92120 Montrouge (FR); LUCIANI, Nathalie, F-78700 Conflans-Saint-Honorine (FR); LE VISAGE, Catherine, F-75018 Paris (FR); GAZEAU, Florence, F-94270 Le Kremlin Bicêtre (FR); WILHELM-HANNETEL, Claire, F-78140 Vélizy (FR)
(74) Mandataire: Regimbeau
(86) Numéro de dépôt international: PCT/EP2012/067048
(87) Numéro de publication internationale: WO 2013/030393

(56) Documents cités:
- US-A1- 2005 074 477
- ROBERT DAMIEN ET AL: "Magnetic micro-manipulations to probe the local physical properties of porous scaffolds and to confine stem cells", BIOMATERIALS, vol. 31, no. 7, mars 2010 (2010-03), pages 1586-1595, XP002674554,
- SCHAEFER R ET AL: "Transferrin receptor upregulation: In vitro labeling of rat mesenchymal stem cells with superparamagnetic iron oxide", RADIOLOGY, RADIOLOGICAL SOCIETY OF NORTH AMERICA, OAK BROOK,IL, US, vol. 244, no. 2, 1 août 2007 (2007-08-01), pages 514-523, XP008094084, ISSN: 0033-8419, DOI: 10.1148/RADIOL.2442060599
- HIROKAZU AKIYAMA ET AL: "Fabrication of complex three-dimensional tissue architectures using a magnetic force-based cell patterning technique", BIOMEDICAL MICRODEVICES, KLUWER ACADEMIC PUBLISHERS, BO, vol. 11, no. 4, 12 février 2009 (2009-02-12), pages 713-721, XP019727805, ISSN: 1572-8781, DOI: 10.1007/S10544-009-9284-X
- LINO MASAAKI: "Effects of homogenous magnetic field on erythrocyte sedimentation and aggregation", BIOELECTROMAGNETICS, vol. 18, no. 3, 1997, pages 215-222, XP002674555, ISSN: 0197-8462
- TESTORF MARTIN F ET AL: "Melanophore aggregation in strong static magnetic fields", BIOELECTROMAGNETICS, vol. 23, no. 6, septembre 2002 (2002-09), pages 444-449, XP002674556, ISSN: 0197-8462
- BRATT-LEAL ANDRES M ET AL: "Magnetic manipulation and spatial patterning of multi-cellular stem cell aggregates", INTEGRATIVE BIOLOGY, vol. 3, no. 12, 10 novembre 2011 (2011-11-10), pages 1224-1232 URL, XP002674557,

## Description

### DOMAINE DE L'INVENTION

L'invention concerne le domaine de la thérapie cellulaire, de la médecine régénératrice et de l'ingénierie tissulaire. Un procédé permettant une agrégation optimale de cellules, typiquement de cellules souches, favorisant leur organisation et avantageusement leur différenciation en particulier dans le contexte de la formation d'un substitut tissulaire, est ainsi décrit. Ce procédé comprend l'exposition à un champ magnétique de cellules souches prétraitées et permet l'obtention d'agrégats cellulaires comprenant des cellules différenciées, en particulier d'agrégats cellulaires de grandes tailles. De façon surprenante et contrairement à la pratique actuelle, ce procédé d'agrégation peut avantageusement être mis en oeuvre en l'absence de matrice de soutien ou support d'adhésion cellulaire tridimensionnel exogène et/ou de facteur de croissance. Sont également décrits les agrégats cellulaires susceptibles d'être obtenus à l'aide d'un tel procédé ainsi que leurs utilisations en tant qu'amorces tissulaires en vue de l'obtention d'une structure tissulaire d'intérêt *in vitro, ex vivo* ou *in vivo,* mais aussi les structures tissulaires ainsi obtenues et leurs utilisations en recherche ou en thérapie en tant que substituts tissulaires. La présente description fournit en outre une méthode permettant avantageusement de suivre le développement du tissu d'intérêt *in vivo.*

### ETAT DE LA TECHNIQUE

La méthode d'agrégation cellulaire couramment employée en ingénierie tissulaire consiste à centrifuger lesdites cellules. Cette méthode ne permet toutefois d'obtenir que des agrégats de petites tailles (environ 1 mm³) contenant un nombre limité de cellules (au plus environ 250 000 cellules). Même en augmentant le nombre de cellules centrifugées il n'est pas possible à ce jour d'obtenir des agrégats de volume supérieur, les cellules présentes au coeur de l'agrégat se nécrosant (vraisemblablement du fait de l'accès restreint aux nutriments et à l'oxygène).

La taille limitée des agrégats obtenus n'est pas sans poser de problèmes pour les implantations de routine de substituts tissulaires en clinique. Cette contrainte impose en effet la reconstitution d'une amorce tissulaire présentant une taille critique avant implantation, par mise en culture *ex vivo* du ou des agrégats cellulaires de petites tailles, ou l'implantation d'un nombre très élevé d'agrégats cellulaires pour combler une lésion (surface variable pouvant couramment atteindre 16 cm² dans le cas de lésions du cartilage articulaire). Une telle implantation, en plus de rendre plus délicate la manipulation du chirurgien, est souvent à l'origine de problèmes de rétention desdites amorces au site d'implantation de sorte que seul un faible nombre des cellules implantées participe effectivement à la régénération tissulaire. L'emploi d'une matrice de soutien pour combler la lésion au site d'implantation et favoriser la rétention des agrégats cellulaires s'avère très souvent indispensable dans ce contexte.

L'implantation d'agrégats cellulaires est une stratégie thérapeutique employée en particulier pour le traitement de pathologies du cartilage. Le cartilage joue un rôle essentiel de résistance aux chocs mais possède une capacité très limitée à se régénérer spontanément après une lésion (chocs traumatique, arthrose, etc.). Il est constitué d'une matrice extracellulaire qui lui confère sa résistance mécanique, et de cellules, les chondrocytes, responsables de la synthèse de cette matrice.

Le traitement des pathologies du cartilage, en particulier du cartilage articulaire, se fait, à l'heure actuelle, par des techniques purement chirurgicales (microfacture) ou par des techniques incluant l'implantation de chondrocytes [transplantation ostéochondrale (OATS ou mosaicplasty), implantation/transplantation de chondrocytes autologues ACI/ACT].
La microfracture permet de générer un afflux de sang qui va stimuler une réponse cicatricielle. Les résultats obtenus sont toutefois mitigés et le cartilage néoformé, de nature fibreuse, se dégrade ultérieurement (souvent dans les six à douze mois). Cette méthode est néanmoins utilisée par nombre de chirurgiens orthopédiques car elle est simple à mettre en oeuvre. Elle s'applique aux sujets jeunes (notamment les sportifs) mais n'est pas adaptée aux personnes souffrant d'arthrose chez lesquelles les capacités de régénération du cartilage sont diminuées.
La transplantation ostéochondrale consiste en allogreffes ou autogreffes de cartilage et d'os sous-jacent (un greffon d'os s'attache assez naturellement à un autre morceau d'os alors qu'il faut suturer le cartilage avec un risque de détachement non négligeable de ce dernier). Dans ce dernier cas, du cartilage est prélevé dans les zones où la charge est faible pour être réimplanté dans des zones où la charge est importante. Les principaux problèmes rencontrés sont la morbidité au site donneur (destruction du cartilage sain) et la régénération incomplète et temporaire du fait de la dégénérescence du greffon inadapté aux sollicitations mécaniques du site d'implantation.
Dans le cas des allogreffes (greffons prélevés sur des cadavres et éventuellement conservés après congélation ou lyophilisation), de bons résultats cliniques ont été obtenus sur le long terme (10 ans). Le cartilage est peu sujet aux phénomènes de rejet. Les principales limitations sont la faible disponibilité de greffons et le risque de transmission de maladies.
L'implantation de chondrocytes autologues, possible depuis 1968, est précédée par une biopsie de cartilage sain (Carticel® de la société Genzyme, ACI-Maix® de la société Matricel). Les chondrocytes sont extraits et amplifiés *in vitro* (2D) avant d'être implantés. Un morceau de tissu, suturé au niveau du défaut, assure que les cellules restent localisées au site d'implantation. Le tissu implanté finit toutefois par se dégrader tout comme dans le cas des autogreffes.

Les voies les plus récemment explorées pour l'ingénierie de tissus cartilagineux consistent (i) en l'utilisation de matrices poreuses destinées à favoriser, après leur implantation chez le patient, la migration des cellules différenciées natives dudit patient (US 6,179,871 ; Chondrotissue® de la société BioTissue Technologies ; Robert et al., Biomaterials 31, 2010), ou de matrices combinées à des cellules dont l'action doit être de promouvoir et de guider la régénération tissulaire après l'amplification en culture cellulaire 2D desdites cellules (MACI® de la société Genzyme), (ii) la mise en oeuvre de méthode impliquant une étape de culture en suspension (US 2005/0074477) et (iii) l'implantation de cellules souches mésenchymateuses ou CSM (au stade d'essais cliniques).
Cette dernière voie constitue une variante de l'implantation de chondrocytes. Elle présente toutefois le même problème de rétention des cellules au site d'implantation, auquel s'ajoutent ceux liés à la différenciation des CSM.
Dans ce type de procédure, les CSM sont i) implantées sans traitement préalable, ii) implantées après avoir été activées par des protéines dont le rôle est de les programmer pour devenir des cellules différenciées, ou iii) différenciées *in vitro* et implantées sous forme d'amorces tissulaires.

En la matière, la technique dite « d'agrégation cellulaire par centrifugation » permet la différenciation *in vitro* de cellules souches, telles que les cellules souches mésenchymateuses (CSM), en chondrocytes capables de produire la matrice extracellulaire du tissu cartilagineux (ce processus de différenciation est aussi appelé chondrogenèse). La centrifugation de CSM à 200g pendant 5 minutes, suivie de la culture de l'agrégat cellulaire obtenu en présence de facteurs solubles spécifiques induisant la chondrogenèse pendant 14 jours (cf. Schäler et al., Radiology : vol. 211, n°2, 2007) ou 28 jours, en particulier en présence de TGFβ1, le TGFβ2, le TGFβ3 ou BMP-2, sont des exemples de mise en oeuvre de la technique d'agrégation cellulaire par centrifugation.
Appliquée aux cellules souches destinées à se différencier en cellules cartilagineuses, la méthode d'agrégation cellulaire par centrifugation provoque toutefois la formation d'une coque fibreuse autour de l'agrégat cellulaire limitant, dans le cadre d'un usage en clinique dudit agrégat en tant qu'amorce tissulaire, sa capacité d'intégration au tissu cartilagineux natif du sujet receveur et favorisant sa dégradation ultérieure.

Les inventeurs décrivent à présent une solution aux multiples problèmes rencontrés lors de la mise en oeuvre des techniques développées dans l'art antérieur telle que la méthode d'agrégation de cellules par centrifugation classiquement utilisée en thérapie cellulaire.
Cette solution permet l'obtention de structures cellulaires comprenant des cellules différenciées, en particulier de structures cellulaires de grandes tailles, ce qui permet l'implantation d'un nombre restreint de telles structures au niveau de la lésion et facilite leur manipulation par le chirurgien. Elle permet en outre de s'affranchir de l'utilisation de toute matrice de soutien.

### RESUME DE L'INVENTION

Les inventeurs décrivent un procédé d'agrégation de cellules, en particulier de cellules souches et/ou de cellules en voie ou en cours de différenciation, comprenant:
a) la mise en contact *in vitro* ou *ex vivo* de cellules souches ou de cellules en voie de différenciation avec des particules magnétiques, ladite mise en contact permettant l'obtention de cellules magnétisées, et
b) l'exposition desdites cellules magnétisées à un champ magnétique, ladite exposition permettant la formation d'un agrégat cellulaire.

Le procédé selon l'invention permet une agrégation optimale des cellules, typiquement de cellules souches, et favorise leur organisation et avantageusement leur différenciation.

Les inventeurs décrivent également l'agrégat cellulaire susceptible d'être obtenu à l'aide d'un tel procédé, ledit agrégat comprenant des cellules magnétisées, par exemple des cellules souches magnétisées, des cellules souches magnétisées en cours de différenciation, des cellules magnétisées en cours de différenciation, et/ou des cellules magnétisées différenciées. Ils décrivent aussi l'utilisation d'un ou plusieurs de ces agrégats cellulaires, ou le résultat de la fusion desdits agrégats, en tant qu'amorces tissulaires injectables ou implantables *in vivo* chez l'animal.

Les inventeurs décrivent par ailleurs un procédé de préparation d'une structure tissulaire comprenant la mise en oeuvre d'un procédé d'agrégation de cellules tel que décrit dans le présent texte et, éventuellement, une étape de culture d'un ou de plusieurs agrégats cellulaires obtenus à l'issue dudit procédé d'agrégation, ainsi que la structure tissulaire susceptible d'être obtenue à l'aide d'un tel procédé, ladite structure tissulaire comprenant (i) des cellules magnétisées, par exemple des cellules souches magnétisées, des cellules souches magnétisées en cours de différenciation, et/ou des cellules magnétisées en cours de différenciation, ainsi que éventuellement des cellules magnétisées différenciées, lesdites cellules étant matures ou immatures, et identiques ou différentes, et (ii) une matrice extracellulaire. La description concerne aussi l'utilisation de cette structure tissulaire en tant que substitut tissulaire, un tel substitut pouvant être implanté *in vivo.*

La technologie décrite permet en particulier de lever le verrou technique de la taille limitée des amorces tissulaires puisqu'elle rend possible l'obtention d'agrégats comprenant un nombre de cellules 10 fois supérieur à celui des agrégats obtenus à l'aide des techniques existantes, et donc d'amorces tissulaires implantables de taille modulable, en particulier de grandes tailles, exploitables en clinique. Cette augmentation de taille des agrégats différenciés résulte vraisemblablement de la géométrie initiale obtenue grâce au procédé d'agrégation décrit favorable aux échanges entre ledit agrégat et le milieu environnant. Les inventeurs révèlent pour la première fois que les agrégats cellulaires ainsi obtenus en plus d'être viables ne perdent pas leurs propriétés de fusion à long terme. Ils peuvent ainsi fusionner entre eux tout au long du processus de chondrogenèse. Les agrégats cellulaires peuvent en outre avantageusement être cultivés en grand nombre dans un seul bioréacteur contrairement aux agrégats existants de petites tailles qui requièrent l'utilisation d'un tube par agrégat.
La présente invention rend possible l'organisation des agrégats cellulaires dans l'espace pour obtenir n'importe quelle forme, en particulier celle du site de lésion, de même que la coculture de plusieurs types cellulaires organisés, par exemple de cellules cartilagineuses et de cellules osseuses.
Le procédé décrit, applicable à grande échelle, permet en outre d'obtenir un tissu fonctionnel sans avoir besoin de lui associer une matrice de soutien (toujours problématique eut égard aux questions de biocompatibilité et de dégradation) et permet de s'affranchir en partie voire en totalité de l'utilisation de facteurs de croissance coûteux. Les substituts tissulaires, obtenus dans le contexte de la présente invention, de même que les agrégats cellulaires décrits obtenus par les inventeurs, sont en effet avantageusement implantables de manière précoce, c'est-à-dire avant la fin du processus de différenciation.

Par ailleurs, de part l'incorporation de particules magnétiques au sein de la structure tissulaire, le procédé selon l'invention permet le suivi non-invasif du développement *in vivo* de cette structure après son implantation, par exemple à l'aide de la technique d'imagerie par résonance magnétique (IRM) bien connue de l'homme de l'art.

### BREVE DESCRIPTION DES FIGURES

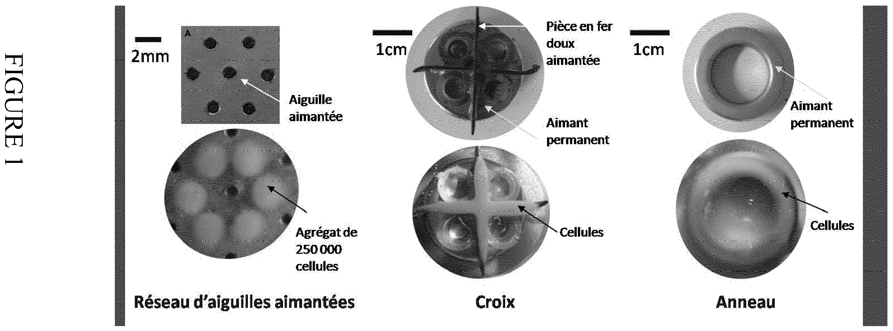

**Figure 1** : Dispositifs magnétiques
   La figure 1 montre des exemples de dispositifs pouvant être utilisés pour la condensation magnétique de cellules dans le cadre de la présente invention. De gauche à droite : réseau hexagonal d'extrémités d'aiguilles aimantées tronquées amenant, par exemple, à la formation d'agrégats de 250 000 cellules au-dessus de chaque extrémité ; croix en fer doux, aimantée par un aimant permanent, amenant à la formation d'un agrégat de 4 millions de cellules en forme de croix ; aimant permanent en forme d'anneau, amenant à la formation d'un agrégat cellulaire de 4 millions de cellules de même géométrie.

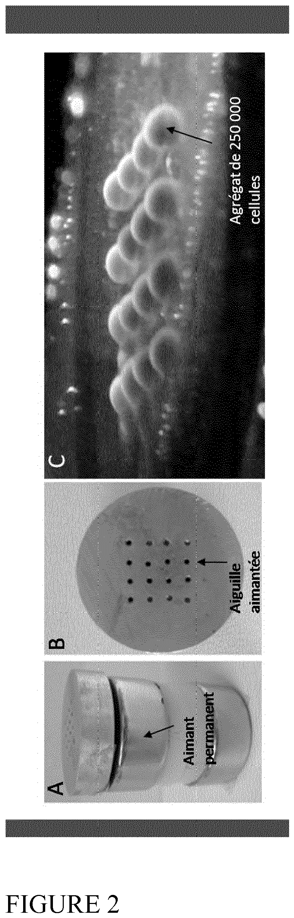

**Figure 2** : Bioréacteur 3D
   La figure 2 donne un exemple de culture en masse d'agrégats cellulaires. La géométrie utilisée est ici un réseau carré d'extrémités d'aiguilles tronquées (Figure 2B), aimantées grâce à un aimant permanent placé en-dessous de ce réseau (Figure 2A). Dans cet exemple, 16 agrégats de 250 000 cellules chacun peuvent être cultivés simultanément (Figure 2C).
**Figure 3** : Condensation magnétique pour l'obtention d'amorce de cartilage de grande taille.
   Cette figure illustre l'intérêt de la condensation magnétique pour l'obtention de tissu cartilagineux par différenciation de cellules souches.
   Figure 3A : un dépôt initial d'un ou de plusieurs millions de cellules selon une géométrie en ligne ou en croix aboutit à la formation d'agrégats sphériques. Lors de cette transition de géométrie, l'agrégat cellulaire maintient une surface d'échange importante avec le milieu extracellulaire, favorable à la diffusion des nutriments et de l'oxygène au coeur de l'agrégat (flèches pointant vers les espaces vacants).
   Figure 3B : le volume final des agrégats obtenus par cette technique est largement supérieur à celui des agrégats obtenus par la technique classique de confinement par centrifugation.

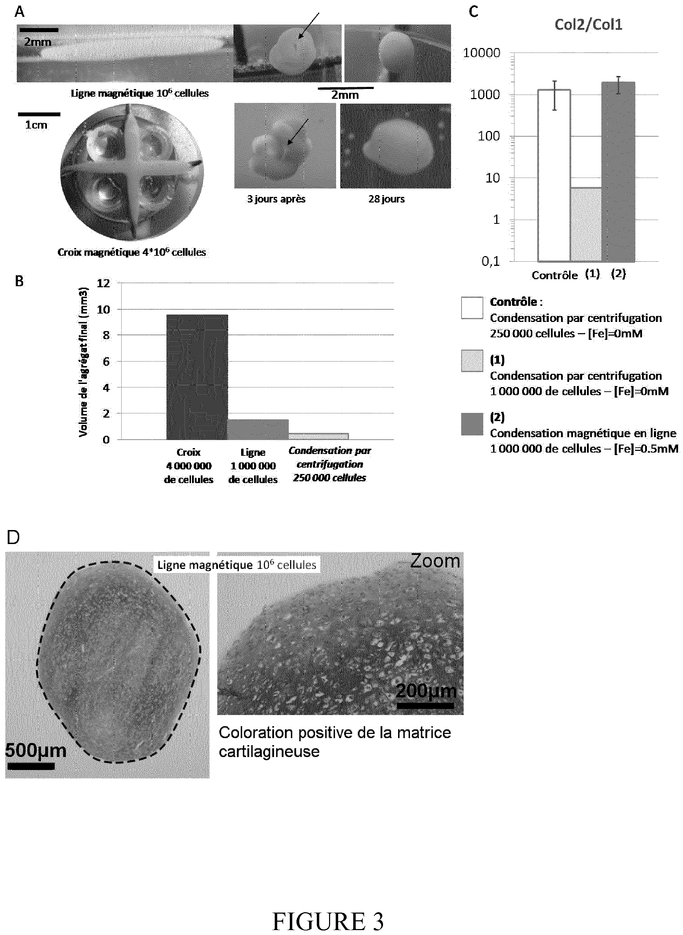

   Figure 3C : la différenciation des cellules souches peut être évaluée par quantification de l'expression du gène codant pour le collagène II rapportée à celle du gène codant pour le collagène I. Sur ce graphique, on observe que l'augmentation du nombre de cellules confinées par centrifugation de 250 000 à 1 million conduit à une diminution drastique de l'expression des gènes de la matrice cartilagineuse (comparaison du contrôle avec (1)). Au contraire, lorsqu'un million de cellules sont confinées par condensation magnétique, l'expression de ces mêmes gènes reste comparable à celle dans le cas d'un confinement par centrifugation de 250 000 cellules (comparaison du contrôle avec (2)) et est plus de 100 fois supérieure à celle dans le cas d'un confinement par centrifugation d'un million de cellules (comparaison (1) et (2)).
   Figure 3D : L'analyse histologique du tissu obtenu à partir de la ligne magnétique (un million de cellules sur un centimètre) montre une coloration positive de la matrice cartilagineuse (cercle en pointillé).
**Figure 4** : Assemblage d'amorces de cartilage par fusion.
   Figure 4A : Les agrégats formés par condensation magnétique possèdent une capacité intrinsèque à fusionner comme le montre la formation de « ponts tissulaires » (flèches) après la simple mise en contact de ces agrégats.
   Figure 4B : Cette propriété s'avère intéressante dans une approche « bottom up » où l'on cherche à former des substituts tissulaires de cartilage de taille et de géométrie adaptées au défaut du patient. Des agrégats peuvent être assemblés par fusion pour former des « sticks » de cartilage, eux-mêmes assemblés en feuillets avant que ces feuillets fusionnent en cartilage épais.
   Figure 4C : Cette image montre la fusion de 16 unités de 200 000 cellules, 4 agrégats parmi les 16 initiaux étant désignés par les flèches.
   Figure 4D : L'analyse en histologie d'un agrégat (stick de cartilage) obtenu par fusion de deux unités révèle une coloration positive de la matrice cartilagineuse (cercles en pointillés).
   Figure 4E: La fusion séquentielle de seize unités permet l'obtention d'un tissu continu (feuillet) de grande taille. Par ailleurs, l'analyse histologique du tissu formé révèle une coloration positive de la matrice cartilagineuse (surface délimitée par les pointillés).
**Figure 5** : Visualisation d'un agrégat de cartilage obtenu par condensation magnétique.
   La présence de nanoparticules magnétiques dans l'agrégat le rend visible par imagerie IRM volumique et non invasive. Les images ont été prises avec un appareil IRM Brucker 4.7T, en utilisant une séquence 3D écho de gradient (pondérée en susceptibilité magnétique) avec TR/TE = 20/5ms, angle de renversement= 25°, résolution spatiale 50x50x50µm. Les agrégats de cartilage observés ont été formés par condensation magnétique à partir de 250 000 cellules souches rendues magnétiques (A : marquage [Fe]=0.05mM, 30min, 1 pg de fer par cellule et B : marquage [Fe]=0.1mM, 30min, 2 pg de fer par cellule) de nanoparticules magnétiques, et mises en culture pendant 28 jours.

### DESCRIPTION DETAILLEE DE L'INVENTION

Les inventeurs ont développé un procédé améliorant de manière très significative l'organisation des cellules dans le cadre d'un processus d'agrégation cellulaire capable de manière très avantageuse de favoriser la différenciation des cellules, en particulier des cellules en voie de différenciation et/ou des cellules souches, typiquement des cellules souches mésenchymateuses (CSM), des cellules souches embryonnaires (ES) ou des cellules reprogrammées (« induced Pluripotent Stem Cells » ou cellules iPSC, encore identifiées dans le cadre de la présente description par l'expression « cellules progénitrices induites »), en appliquant auxdites cellules la technique dite « d'agrégation cellulaire par magnétisation ». Cette technique comprend (i) l'introduction de particules magnétiques dans les cellules (par internalisation spontanée), puis (ii) la formation et l'organisation d'agrégats cellulaires de formes géométriques contrôlées par exposition des cellules magnétisées à un champ magnétique externe. L'étape (i) de ce procédé peut être mise en oeuvre *in vitro* ou *ex vivo.* L'étape (ii) peut être réalisée *in vitro*, *ex vivo* ou *in vivo* au choix de l'homme du métier, de préférence dans des conditions adaptées à l'application ultérieure envisagée de l'agrégat cellulaire, par exemple de préférence dans des conditions adaptées au stade du processus de différenciation auquel se trouvent les cellules et/ou au stade de différenciation cellulaire que l'on souhaite obtenir. Un tel procédé offre des perspectives cliniques inenvisageables auparavant compte tenu des contraintes techniques, expliquées précédemment, associées aux méthodes existantes.

Les inventeurs décrivent ainsi un procédé d'agrégation de cellules comprenant :
a) la mise en contact *in vitro* ou *ex vivo* de cellules, en particulier de cellules souches ou de cellules en voie de différenciation avec des particules magnétiques, ladite mise en contact permettant l'obtention de cellules magnétisées, et
b) l'exposition desdites cellules magnétisées à un champ magnétique, de préférence focalisé, ladite exposition permettant la formation d'un agrégat cellulaire.

La méthode d'agrégation décrite s'applique à tous les types cellulaires pouvant être rendus magnétiques et de préférence susceptibles de suivre une voie de différenciation. Les cellules susceptibles d'être utilisées sont typiquement des cellules animales, de préférence des cellules de mammifères, encore plus préférentiellement des cellules d'êtres humains. Ces cellules peuvent être choisies par exemple parmi des cellules souches, des cellules en cours de différenciation, ou des cellules reprogrammées, typiquement des cellules progénitrices induites. Il peut s'agir de cellules d'origine embryonnaire, foetale, néo-natale et/ou adulte, ainsi que de cellules matures ou immatures. Il peut s'agir par exemple de cellules provenant du sang, de la moelle épinière, du placenta, de l'os, du cartilage, du muscle, de la peau ou du tissu adipeux. Des exemples de cellules utilisables dans le contexte de l'invention comprennent (sans s'y restreindre) : cellules souches adultes d'origine mésenchymateuse (provenant typiquement de la moelle osseuse ou du tissu adipeux), cellules souches embryonnaires, cellules progénitrices induites, cellules progénitrices du sang périphérique, cellules progénitrices du sang de cordon ombilical ou du système musculo-squelettique (par exemple cellules précurseurs myogéniques ou cellules souches mésenchymateuses). Les cellules souches susceptibles d'être utilisées en tant qu'outils de thérapie cellulaire dans le cadre de l'invention sont d'origine embryonnaire [cellules souches (CS) embryonnaires (CSE)] ou non embryonnaire (tissus foetaux, néonataux et adultes), et proviennent d'un animal, typiquement d'un mammifère, de préférence d'un être humain.
Les cellules souches embryonnaires (CSE) ont, *in vitro,* un potentiel de multiplication infini et peuvent donner naissance à tous les types de cellules. De telles cellules peuvent être obtenues sans destruction de l'embryon dont elles sont issues par exemple à l'aide de la technique décrite par Chung et al. (cf. Cell Stem Cell. 2008 Feb 7 ; 2(2) : 113-7). L'utilisation de telles cellules souches est particulièrement recommandée pour l'obtention de cardiomyocytes.
Les cellules souches non embryonnaires peuvent être des cellules souches mésenchymateuses (CSM), des cellules progénitrices induites, des cellules souches neuronales, des progéniteurs endothéliaux, etc. Elles sont par exemple issues du synovium, du peristeum, de la moelle osseuse, du tissu adipeux, du sang de cordon ombilical, du placenta, de la gelée de Wharton, etc.
Des cellules souches non embryonnaires préférées, susceptibles d'être utilisées dans le cadre de l'invention, sont choisies parmi les cellules souches adultes d'origine mésenchymateuse ou CSM, les cellules stromales (en particulier pour l'obtention de chondrocytes, hépatocytes ou cardiomyocytes).
Certains types de cellules souches non embryonnaires présentent l'avantage de pouvoir être prélevés facilement et transplantés chez un même individu (autogreffe) éliminant ainsi tout risque de rejet. Ainsi, les CSM peuvent par exemple être prélevées par ponction de moelle osseuse chez le patient puis isolées par attachement spontané sur un support en plastique avant d'être utilisées dans le cadre de la présente invention. D'autres sources de CSM sont par exemple le tissu adipeux, le synovium et le péristeum.

La reprogrammation génétique de cellules différenciées adultes (cf. Yu J, Vodyanik MA, et al. (2007). "Induced Pluripotent Stem Cell Lines Derived from Human Somatic Cells". Science 318 (5858): 1917-1920; et Takahashi K, et al. (2007). "Induction of Pluripotent Stem Cells from Adult Human Fibroblasts by Defined Factors". Cell 131 (5): 861-872.) permet également d'obtenir des cellules pluripotentes aux mêmes capacités de multiplication et de différenciation que les cellules souches embryonnaires. Ces cellules, classées dans le cadre de la présente description dans la catégorie des cellules souches, sont appelées iPSC (induced Pluripotent Stem Cells) ou cellules progénitrices induites et sont également, comme indiqué précédemment, utilisables dans le cadre de la présente invention.

La mise en contact *in vitro* ou *ex vivo* de cellules avec des particules magnétiques permet l'obtention de cellules dites magnétisées selon la technique dite de « marquage magnétique » ou de « magnétisation ». Cette étape, classiquement mise en oeuvre par incubation des cellules avec les particules magnétiques, est une étape nécessaire dans la mise en oeuvre du procédé revendiqué et précède l'étape dite « d'agrégation magnétique » qui implique d'exposer à un champ magnétique les cellules dont on souhaite l'agrégation.

La magnétisation des cellules repose sur la fixation de particules magnétiques à la surface des cellules à magnétiser ou sur l'internalisation de particules magnétiques par lesdites cellules. Le mécanisme d'internalisation des nanoparticules par les cellules repose sur l'invagination de la membrane cellulaire suivie de la formation de vésicules d'endocytose remplies de particules magnétiques. Ces vésicules transportent leur contenu au sein du cytoplasme pour aller le délivrer dans les endosomes tardifs ou lysosomes.

Un exemple de procédé de magnétisation de cellules par internalisation de nanoparticules magnétiques est décrit dans la publication Wilhelm C. et Gazeau F. (Biomaterials. 2008; August 29(22):3161-3174). Dans ce cas, l'étape d'incubation est alors suivie d'une période dite de « chasse » dans du milieu de culture cellulaire sans nanoparticules pour permettre l'internalisation complète de toutes les nanoparticules initialement adsorbées à la surface des cellules. On observe qu'une à deux heures après le début de l'incubation, les particules se retrouvent ainsi concentrées à l'intérieur des cellules, et leur nombre varie entre environ 10⁵ et environ 10⁸ particules par cellules, par exemple 10⁷ particules par cellules (ce qui correspond à environ 10 pg de fer).

Des particules magnétiques utilisables dans le cadre de la présente invention sont des microparticules magnétiques ou des nanoparticules magnétiques, de préférence des nanoparticules magnétiques.
La taille (diamètre moyen) des nanoparticules est inférieure à 1 µm, typiquement comprise entre 5 nm et 999 nm, de préférence comprise entre 5 et 250 nm, de manière particulièrement préférée comprise entre 5 et 50 nm, encore plus préférentiellement entre 5 et 15 nm, et est par exemple de 6, 7, 8, 9 ou 10 nm. Les petites particules (dont la taille est typiquement inférieure à 50 nm) auront plus facilement tendance à être internalisées dans les cellules que les grosses particules.
La taille (diamètre moyen) des microparticules est supérieure ou égale à 1 µm, typiquement comprise entre 1 µm et 4 µm, de préférence entre 1 µm et 2 µm.

Les particules utilisables dans le cadre de la présente invention sont des particules magnétiques, i.e., des particules constituées d'au moins un matériau magnétique, des particules comprenant plusieurs matériaux magnétiques, et des particules comprenant au moins un matériau magnétique associé ou non à d'autres matériaux non magnétiques (particules hybrides). Des exemples de particules hybrides utilisables sont les nanoparticules de type coeur-coquille, les particules dissymétriques (de type Janus, ou des structures poreuses ou mésoporeuses (par exemple en association avec de la silice).

Le matériau magnétique peut être dur ou doux, c'est-à-dire de forte ou faible anisotropie magnétique.

Des exemples de matériaux magnétiques utilisables dans le cadre de la présente invention sont les matériaux ferromagnétiques, ferrimagnétiques, superparamagnétiques, antiferromagnétiques, ou éventuellement paramagnétiques.
Un matériau ferrimagnétique utilisable dans le cadre de la présente invention peut être par exemple une ferrite de structure spinelle, par exemple choisi parmi maghémite, magnétite, ferrite de nickel, ferrite de cobalt, etc.
Un matériau paramagnétique utilisable dans le cadre de la présente invention peut être choisi par exemple parmi un matériau dopé au gadolinium, un oxyde de gadolinium ou un oxyde de manganèse.
Un exemple de particule constituée de matériau superparamagnétique utilisable dans le cadre de la présente invention peut être une nanoparticule de ferrite (oxyde de fer (maghémite par exemple) dont la taille est de préférence inférieure à 20 nm. L'utilisation de matériaux ferromagnétiques ou ferrimagnétiques est préférée. Des exemples particulièrement préférés de matériaux ferro- ou ferrimagnétiques sont les oxydes de fer tels que la magnétite (Fe₃O₄) ou la maghémite (γFe₂O₃).

Le matériau choisi peut également être constitué d'oxyde métallique (oxyde de fer, oxyde de cobalt, oxyde de nickel, oxyde de manganèse, oxyde de platine), de métaux purs (par exemple fer, cobalt, nickel) ou d'alliages de métaux (par exemple CoPt, FePt), en association ou non avec des métaux non-magnétiques (par exemple l'argent ou l'or).

Les particules magnétiques utilisées dans le cadre de la présente invention peuvent se présenter sous une forme brute, être liées à un ou à plusieurs ligands (citrate par exemple) ou polymères (dextran ou polyethylene glycol (PEG) par exemple), être partiellement ou totalement recouvertes ou enrobées de tels ligands ou de tels polymères, ou encore être encapsulées par exemple dans des micelles, des nanotubes de carbone ou de titane, des liposomes, des vésicules cellulaires, des vecteurs viraux, à l'aide de techniques bien connues de l'art.

Des doses de particules efficaces dans le cadre de la présente invention, facilement déterminables par l'homme du métier, sont par exemple d'environ 10 pg de fer par cellule (soit environ 0.1 mg de fer pour environ 10 millions de cellules). De telles doses sont environ mille fois inférieures à celles utilisées en imagerie (20-50 µmoles/kg, soit environ 0.1 g de fer pour un patient). A titre de comparaison, la masse totale de fer dans le corps humain est de l'ordre de 3-4 g tandis que 1 à 2 mg sont renouvelés chaque jour. Les études pratiquées jusqu'à présent sur les nanoparticules d'oxyde de fer classiquement utilisées dans le cadre de la technique de magnétisation n'ont révélé aucun caractère toxique. Des nanoparticules constituées d'oxyde de fer ont par ailleurs été approuvées en tant qu'agents de contraste utilisables en Imagerie par Résonnance Magnétique (IRM) pour une utilisation en clinique.
Dans le cadre de leurs expériences, les inventeurs ont pratiqué, de manière systématique, des tests de cytotoxicité et fonctionnalité sur les particules utilisées. Ils ont ainsi pu démontrer l'innocuité des particules en particulier sur la prolifération et la différenciation cellulaire. L'usage de telles particules s'avère ainsi particulièrement intéressant chez l'animal, les mammifères en particulier, l'être humain de préférence.

L'homme du métier est par ailleurs en mesure de maîtriser, par les conditions de mises en oeuvre des méthodes décrites dans le cadre de la présente invention, les quantités de nanoparticules internalisées par les cellules, notamment par les cellules souches, et donc d'appliquer un champ magnétique externe d'intensité modulable, contrôlable à l'échelle de la cellule.
L'incubation, par exemple pendant 30 minutes, par exemple de cellules souches mésenchymateuses humaines avec un milieu contenant des nanoparticules d'oxyde de fer (d'environ 8 nm de diamètre) à des concentrations en fer allant de 0.05 mM à 5 mM conduit à des masses de fer intracellulaire d'environ 1 à 31 pg par cellule. Une incubation de seulement 1 minute est également possible. Cette durée est en effet suffisante pour permettre l'adsorption des nanoparticules sur la membrane cellulaire. En l'absence de période de chasse, ces nanoparticules se localiseront toutefois essentiellement à la surface de la membrane cellulaire.

La formation, décrite par les inventeurs, d'agrégats cellulaires denses à partir des cellules magnétisées est obtenue à l'aide d'un champ magnétique, avantageusement focalisé, ou de plusieurs champs magnétiques, de préférence focalisés et/ou caractérisés par un important gradient de champs magnétiques (force magnétique cellulaire intense par exemple comprise entre environ 0.1 nN et environ 10 nN).
Ces champs magnétiques sont typiquement générés par des aimants ou par des pièces aimantables. Les champs magnétiques forts peuvent en particulier être générés par exemple par des aimants permanents (matériaux magnétiques durs constitués par exemple de néodymium, nickel, fer, tec.) ; par des pièces aimantées par un aimant permanent placé à proximité ; par des électroaimants (pièces en cuivre ou en or par exemple).
Ces aimants peuvent être usinés aux dimensions et à la géométrie souhaitée. A chaque fois, plusieurs de ces aimants peuvent être combinés pour créer une plus grande variété de formes ou motifs.

Les techniques classiques de microfabrication connues de l'homme de l'art peuvent être mises en oeuvre pour déposer de manière contrôlée sur un substrat (i) par exemple un matériau ferromagnétique de manière à réaliser un aimant permanent ou (ii) un matériau conducteur (typiquement un métal tel que l'or ou le cuivre) de manière à réaliser un électroaimant. Dans ce dernier mode de réalisation, les propriétés magnétiques dépendent alors du passage d'un courant électrique dans le matériau conducteur. Le contrôle de l'intensité du courant électrique au cours du temps permet de moduler l'intensité de la force magnétique appliquée.

L'étape b) du procédé d'agrégation décrit qui correspond à l'exposition des cellules magnétisées à un champ magnétique consiste ainsi typiquement en l'exposition desdites cellules à une ou plusieurs formes aimantées.

Ladite forme peut être choisie parmi un point, la pointe ou l'extrémité d'une aiguille tronquée, une ligne, une croix, une étoile, un anneau, un disque, un cercle, ou plusieurs de l'une et/ou l'autre de ces formes, par exemple un ensemble de points ou un ensemble de lignes, de préférence organisées en réseau (par exemple un réseau d'extrémités d'aiguilles tronquées en tant qu'ensemble de points ou un maillage en tant qu'ensemble de lignes). Ladite forme est avantageusement choisie parmi un réseau d'extrémités d'aiguilles tronquées, une ou plusieurs lignes, une croix et un ou plusieurs anneaux, par exemple disposés de manière concentrique.

Dans un mode de réalisation particulier, la pointe d'une aiguille ou l'extrémité d'une aiguille tronquée (par exemple d'environ 1mm de diamètre), constituée d'un matériel magnétique adapté tel que décrit précédemment ou aimantée par un aimant permanent, peut être utilisée pour créer des agrégats ayant la forme d'une demi-sphère. Plusieurs de ces extrémités d'aiguilles tronquées, alors sources magnétiques « ponctuelles », peuvent être réunies afin d'obtenir plusieurs agrégats sur le même support ou substrat. L'organisation des agrégats cellulaires est alors définie par celle des pointes d'aiguilles magnétiques ou des extrémités d'aiguilles magnétiques tronquées.

L'assemblage ou l'association de ces extrémités d'aiguilles peut refléter, comme expliqué précédemment, une organisation régulière (par exemple un réseau carré ou hexagonal, une spirale, etc.) ou irrégulière (reflétant par exemple la géométrie du défaut du patient). L'espacement des aimants peut en effet être aisément modulé par l'homme du métier.

L'espacement choisi entre les aimants permet de contrôler la fusion des agrégats cellulaires obtenus en induisant un contact plus ou moins étroit entre ces derniers. Ainsi par exemple, dans le cas d'un réseau d'aiguilles magnétiques tronquées, l'espacement entre les extrémités des aiguilles est par exemple compris entre 1 et 20 mm. Cet espacement est de préférence d'au moins 1 mm, par exemple de 2, 3, 4, 5, 10 ou 15 mm.

Dans un autre mode de réalisation particulier, des aimants ou des pièces aimantables de géométrie linéaire sont utilisés. Des pièces, par exemple en fer doux, peuvent être utilisées pour former des « lignes » de cellules de largeur et de longueur modulables. Une longueur typique est par exemple comprise entre 0.5 cm et 2 cm, ou entre 1 cm et 2 cm, pour une épaisseur comprise entre 0,1 mm et 1 mm, typiquement d'environ 0.5 mm, par exemple de 0,2, 0,3, 0,4, 0,6, 0,7, 0,8 ou 0,9 mm. Comme pour les aimants ponctuels, plusieurs de ces lignes magnétiques peuvent être combinées pour former par exemple des croix, des étoiles, des maillages ou associations de lignes plus ou moins espacées (entre 0.5 mm et 2 cm, typiquement entre 1 mm et 2 mm, de préférence entre 1 mm et 1,5 mm, par exemple de 1,1, 1,2, 1,3 ou 1,4 mm).

Dans encore un autre mode de réalisation particulier, l'utilisation d'un aimant en forme d'anneau permet d'obtenir un agrégat de cellules de la même géométrie. Plusieurs de ces anneaux peuvent être assemblés comme expliqué précédemment, par exemple de manière concentrique.

Le procédé selon l'invention permet ainsi de créer des motifs tridimensionnels (3D) dont la géométrie et l'organisation spatiale sont modulables et parfaitement déterminables (agrégats cellulaires ponctuels, branchés, en lignes, en réseau carré, en croix, en réseau hexagonal, etc.). Les géométries linéaires obtenues par condensation magnétique sont particulièrement efficaces en ce qu'elles permettent d'augmenter la surface d'échange avec le milieu ambiant et favorisent ainsi la diffusion des nutriments et de l'oxygène.

La densité d'agrégation cellulaire peut être facilement contrôlée par l'homme du métier en faisant varier l'intensité de la force magnétique appliquée qui peut être comprise entre 1 et 5000 piconewtons (pN), par exemple entre 1 et environ 1000 pN, de préférence entre environ 100 et environ 900 pN, de préférence d'environ 200, 300, 400, 500, 600, 700 ou 800 pN. Ces intensités de force ont été en particulier testées pour les cellules souches destinées à une différenciation en chondrocytes, mais aussi pour d'autres types cellulaires tels que les monocytes, les macrophages, les progéniteurs endothéliaux, les cellules endothéliales, les lymphocytes, et les cellules souches mésenchymateuses.

La méthode d'agrégation décrite par les inventeurs peut être mise en oeuvre sur n'importe quel type de support ou substrat, de ceux interagissant faiblement avec les cellules comme le verre, à ceux interagissant fortement en facilitant l'adhésion cellulaire.

Les interactions avec les cellules peuvent également être générées ou favorisées par la nature du matériau choisi constitutif dudit substrat ou par une préparation ou un traitement préalable dudit matériau, par exemple son recouvrement (« coating ») partiel ou total, à l'aide d'une substance favorisant l'adhésion cellulaire. Une telle substance peut être choisie par exemple parmi la gélatine, la fibronectine, une séquence RGD, et des cellules.
Le substrat peut aussi ou autrement être « prétraité » par incubation par exemple dans du sérum.

Le support ou substrat sur lequel peut être organisé une association ou un réseau d'aimants ou de pièces aimantables est de préférence un substrat bidimensionnel, typiquement une surface constituée d'un matériau choisi par exemple parmi le verre, le plastique, les plastiques commerciaux traités pour la culture cellulaire, les polymères organiques et inorganiques, les matrices minéralisées.
Cette surface est de préférence plane. Il s'agit par exemple d'une boîte de culture, par exemple une boîte de culture non traitée au préalable à l'aide d'une substance favorisant l'adhésion cellulaire.
Dans le cas particulier de l'ingénierie tissulaire d'implants ou substituts tissulaires « biphasiques » (i.e., comprenant deux types cellulaires tels que des cellules osseuses et cartilagineuses), ce substrat pourrait correspondre à une ou plusieurs couches d'ostéocytes ou à une matrice ostéoinductive, telle que par exemple une matrice incorporant du phosphate de calcium ou de l'hydroxyapatite.

Le procédé d'agrégation décrit peut également être mis en oeuvre en présence d'une matrice de soutien (substrat tridimensionnel, substrat 3D ou support d'adhésion cellulaire tridimensionnel exogène). Les matrices de soutien tridimensionnelles classiquement employées sont généralement sélectionnées parmi un gel poreux, par exemple un hydrogel ou un gel à base de polysaccharides; un polymère synthétique ; une matrice partiellement minéralisée; un polymère biologique comme le collagène; de l'os, éventuellement de l'os décellularisé. Dans ce cas, les agrégats cellulaires se forment de préférence non pas à la surface de la matrice de soutien mais *in situ*, i.e., dans ladite matrice de soutien. Le procédé décrit est toutefois avantageusement et de préférence mis en oeuvre en l'absence d'une telle matrice de soutien 3D limitant ainsi les risques d'incompatibilité en cas d'implantation chez l'animal.

Le substrat, typiquement le substrat bidimensionnel, est avantageusement placé, dans le cadre du procédé d'agrégation décrit par les inventeurs, à proximité, par exemple au-dessus ou en dessous, de la source magnétique (aimant ou pièce aimantable associée à un aimant permanent). Les cellules magnétisées sont ensuite déposées à proximité de la source magnétique, typiquement sous forme de suspension diluée, sur tout ou partie du substrat, de préférence sur l'ensemble du substrat, ou sous forme de suspension concentrée sur une ou plusieurs parties du substrat. Bien entendu, les cellules magnétisées peuvent selon un autre mode de réalisation de l'invention, être déposées sur le substrat ou à proximité du substrat avant exposition de ce dernier à la source magnétique.

Le nombre de cellules utilisées destinées à former un agrégat varie entre environ 0.1 et environ 4 millions de cellules, par exemple entre environ 300 000 cellules et 3 millions de cellules, typiquement 400 000, 500 000, 600 000, 700 000, 800 000 ou 900 000 cellules, et plusieurs de ces agrégats peuvent être associés ultérieurement. Le nombre de cellules utilisées est par exemple de 0.2, 0.5, 1 et 2 millions de cellules, de préférence de 1 million de cellules.
Que les cellules soient déposées sous forme de suspension diluée ou concentrée, la création quasi instantanée de zones denses en cellules et de zones faiblement cellularisées peut être observée au moment de leur exposition au(x) champ(s) magnétique(s).
La durée d'exposition des cellules souches au champ(s) magnétique(s) pour créer un agrégat présentant une densité cellulaire exploitable en particulier en clinique est comprise entre environ 1 minute et plusieurs dizaines de jours selon la nature des cellules utilisées, le degré de différenciation cellulaire souhaité et/ou la forme souhaitée de l'agrégat. Par exemple, dans le cas de la chondrogenèse, ce temps peut être compris entre 1 minute et la durée complète du cycle de différenciation (28 jours), avantageusement entre 1 minute et 1 heure, de préférence entre 20 min et 1 heure. L'exposition des cellules souches au champ(s) magnétique(s) pendant la durée sélectionnée peut être continue ou transitoire. Elle peut par exemple être transitoire en particulier pendant les 10 à 20 premières minutes de mise en oeuvre de l'étape b) du procédé selon l'invention lorsque cette dernière dure plus d'une heure.

Les facteurs de différenciation (facteurs de croissance) sont des éléments coûteux, essentiels dans les procédés de l'art antérieur actuellement mis en oeuvre, par exemple pour stimuler la chondrogenèse *in vitro.* Ils favorisent entre autres choses l'agrégation des cellules lorsque celle-ci est nécessaire au processus de maturation et de différenciation. Le procédé d'agrégation décrit par les inventeurs permet toutefois de s'affranchir partiellement voire totalement de l'utilisation de tels facteurs, par exemple, typiquement de TGFβ1, TGFβ3 et/ou BMP-2.

Le procédé décrit par les inventeurs permet de créer, par condensation magnétique, des assemblages cellulaires denses capables de différenciation et aux capacités de fusion élevées et prolongées dans le temps qui présentent un potentiel d'intégration élevé *in vivo.* Les agrégats différenciés obtenus (par exemple les amorces de tissu cartilagineux) sont de taille bien supérieure à ceux obtenus à l'aide des techniques connues. Ainsi, le volume final de l'agrégat cellulaire différencié obtenu est multiplié par 10 environ par rapport au volume d'un agrégat obtenu à l'aide des techniques connues. Par exemple pour environ 4 millions de cellules utilisées, le volume final de l'agrégat cellulaire obtenu à l'aide d'une méthode d'agrégation telle que décrite par les inventeurs peut ainsi atteindre voire dépasser les 10 mm³ contre environ 1 mm³ dans les conditions de l'état de l'art. De manière inattendue, les inventeurs ont en effet constaté que la géométrie étalée initiale ne se maintient pas et évolue spontanément et de façon systématique vers une géométrie sphérique (voir Figure 3).

La présente description concerne également les agrégats cellulaires, typiquement les agrégats cellulaires sphériques, susceptibles d'être obtenus à l'aide d'un procédé tel que décrit précédemment, constitués (i) de cellules, de préférence de cellules souches, et éventuellement et avantageusement en outre de cellules différenciées, et/ou de cellules (par exemple de cellules souches) en cours de différenciation, ainsi que éventuellement et avantageusement (ii) d'une matrice extracellulaire (sécrétée par les cellules différenciées), avantageusement dépourvue de coeur nécrotique. Typiquement, les cellules présentes dans l'agrégat sont ou comprennent des cellules magnétisées.

La forme et la taille de l'agrégat obtenu peuvent être adaptées par l'homme du métier, comme expliqué précédemment, en faisant varier la nature et/ou la forme ou géométrie de la source magnétique utilisée, et/ou le nombre de cellules magnétisées exposées à un champ magnétique.
La densité cellulaire de l'agrégat peut être adaptée par l'homme du métier en faisant varier le nombre de cellules formant l'agrégat et/ou l'intensité de la force magnétique s'exerçant sur les cellules. Cette intensité peut elle-même être aisément modulée par l'homme du métier à même de sélectionner en fonction de ses besoins la nature et la quantité de matériel magnétique associé aux cellules, la nature et la forme de la source magnétique, ainsi que la distance entre les cellules et ladite source. L'intensité peut être par exemple modulée comme expliqué précédemment en faisant varier les conditions expérimentales du marquage magnétique.

La présente description concerne également l'utilisation des agrégats cellulaires présentement décrits en tant qu'amorces tissulaires injectables (en suspension) ou implantables *in vivo* chez l'animal, le mammifère en particulier, l'homme de préférence. La taille maximale des agrégats pouvant être injectés en suspension dépend du diamètre de l'aiguille utilisée, lui-même lié au site d'injection.
Ces agrégats cellulaires peuvent également et avantageusement être assemblés (fusion) en structures cellulaires de grandes tailles et de formes contrôlées. Il est en outre possible, dans le contexte de la présente invention, de provoquer la fusion d'agrégats cellulaires différents en ce qu'ils sont constitués de types cellulaires différents (cellules osseuses et cartilagineuses par exemple).

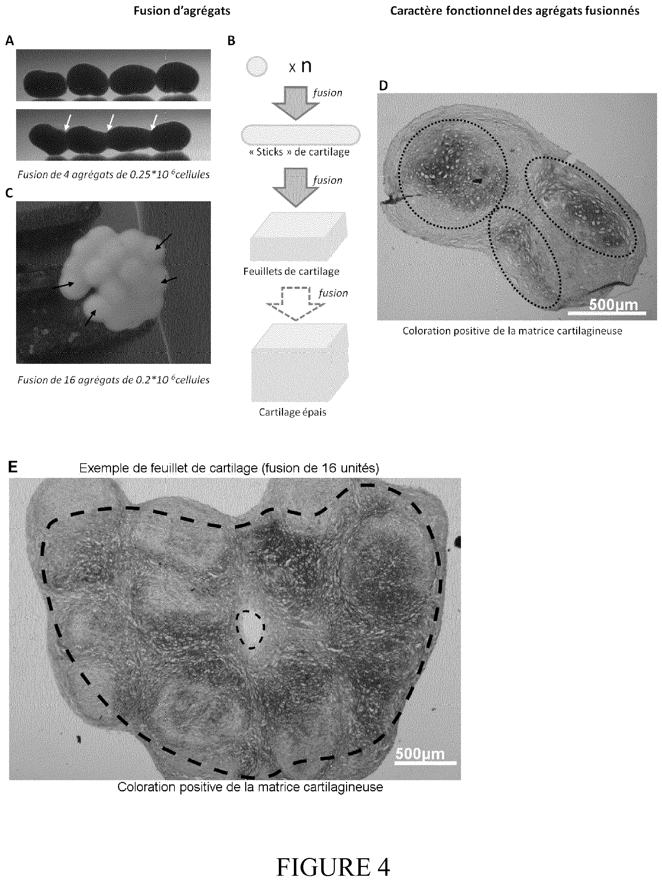

La présente invention concerne ainsi également un procédé de préparation d'une structure tissulaire comprenant la mise en oeuvre d'un procédé d'agrégation de cellules tel que décrit par les inventeurs. Les inventeurs ont notamment démontré, comme expliqué précédemment, que le procédé, dit de condensation magnétique, permettait de s'affranchir de l'étape de centrifugation des cellules pour obtenir des agrégats cellulaires de tailles et formes (ou géométrie) modulables, de préférence de grandes tailles, après leur exposition à un champ magnétique. Il est ainsi possible d'obtenir des bâtonnets (voir la figure 4A par exemple) ou encore des feuillets cellulaires/tissulaires, eux-mêmes assemblables (du fait de leur forte aptitude à fusionner) pour i) augmenter l'épaisseur d'une structure tissulaire ou ii) assembler des structures comprenant des types cellulaires différents (par exemple os et cartilage, cellules souches et cartilage, ou encore cellules souches et os).
Le procédé selon l'invention de préparation d'une structure tissulaire peut comprendre une étape de culture d'un agrégat cellulaire selon l'invention, ou de coculture de plusieurs de ces agrégats cellulaires (constitués de cellules identiques ou différentes). Les agrégats obtenus après exposition à un champ magnétique créé par des micro-aimants ponctuels peuvent être de taille similaire à celle des agrégats obtenus à l'aide du procédé d'agrégation par centrifugation (dit de condensation par centrifugation) (voir Figure 2). L'avantageuse contribution du présent procédé, même pour des agrégats de tailles similaires, est de rendre possible la différenciation cellulaire simultanée jusqu'au stade souhaité d'un très grand nombre d'agrégats dans un même bioréacteur (voir Figure 2C) alors que la technique impliquant une centrifugation impose l'utilisation d'un unique agrégat par tube de centrifugation. Le procédé d'agrégation selon l'invention est ainsi possible et avantageusement réalisé sans étape de centrifugation. Par ailleurs, plusieurs de ces agrégats peuvent fusionner pour donner naissance à des structures de taille supérieure.
L'étape de culture du procédé selon l'invention peut ainsi concerner un agrégat ou simultanément plusieurs agrégats cellulaires cultivés dans un incubateur pendant une durée qui peut être comprise entre une heure et plusieurs jours, voire plusieurs mois. Ainsi, dans le cas de la chondrogenèse, l'étape de culture peut durer entre un jour et 28 jours. Cette étape de culture est généralement réalisée en présence de facteurs de croissance et/ou de stimuli physiques. Ainsi par exemple, l'incubation des agrégats cellulaires dans un milieu chondrogénique contenant un ou plusieurs facteurs de croissance tels ceux mentionnés précédemment, de préférence du TGFβ3, est souhaitable si l'on souhaite obtenir une structure tissulaire cartilagineuse.
Le comportement similaire, lors des premiers jours du processus de différenciation, des agrégats cultivés en présence d'un ou de plusieurs facteurs de croissance induisant la chondrogenèse d'une part et des agrégats cultivés en l'absence de tels facteurs d'autre part, démontre que le procédé selon l'invention de préparation d'une structure tissulaire peut être mis en oeuvre en l'absence de facteur de croissance, de préférence en l'absence de TGFβ1, TGFβ3 et/ou BMP-2, au moins durant l'étape d'agrégation, et avantageusement également durant le premier jour ou les 2, 3, 4, 5, 6, 7 ou 8 premiers jours de l'étape de culture.
Dans un mode de réalisation particulier de la présente invention, la première phase de culture des agrégats est réalisée en l'absence de facteur de croissance et est avantageusement suivie d'une seconde phase de culture en présence de l'un et/ou l'autre des facteurs de croissance habituellement utilisés par l'homme du métier selon le ou les types cellulaires concernés et la nature de la ou des structures tissulaires qu'il souhaite obtenir.
La seconde phase de culture peut aussi comprendre ou autrement consister en l'injection ou l'implantation des agrégats ayant été soumis à la première phase de culture. Dans ce cas, l'environnement *in vivo* prend le relais des facteurs de croissance introduits dans le milieu de culture.

Les cellules agrégées peuvent être, ou non, exposées durant tout ou une partie de l'étape de culture, par exemple 1, 3, 7, 14 ou 28 jours, à un champ magnétique tel que défini précédemment, de façon continue ou séquentielle.

La phase de culture telle que décrite dans le cadre de la présente invention des cellules agrégées ou des agrégats obtenus peut être mise en oeuvre en présence d'un champ magnétique tel que défini précédemment, de façon continue ou séquentielle. Ce champ magnétique peut être créé à l'aide d'une ou de plusieurs sources magnétiques, sources susceptibles d'être rapprochées l'une de l'autre ou les unes des autres comme expliqué plus haut dans la présente description afin de favoriser la fusion des agrégats pour former une structure tissulaire de plus grande taille et de forme souhaitée.

Tout comme le procédé d'agrégation, le procédé de préparation d'une structure tissulaire décrit peut être avantageusement mis en oeuvre en l'absence d'une matrice de soutien tridimensionnelle (3D).

La présente description concerne par ailleurs la structure tissulaire obtenue ou susceptible d'être obtenue à l'aide d'un procédé tel que décrit dans le présent document. Une telle structure comprend (i) des cellules différenciées, par exemple des cellules cartilagineuses, typiquement des cellules magnétisées différenciées, et (ii) une matrice extracellulaire, par exemple une matrice extracellulaire cartilagineuse (composée de fibres de collagène de type II et/ou de protéoglycanes tel que l'aggrégane) si les cellules différenciées présentes sont des cellules cartilagineuses. Elle peut en outre comprendre des cellules souches et/ou des cellules en cours de différenciation. Il s'agit typiquement de cellules magnétisées.
La présente description concerne en outre l'utilisation avantageuse d'une telle structure tissulaire selon l'invention, en tant que substitut tissulaire. Un tel substitut peut être implanté *in vivo* chez l'animal, le mammifère en particulier, l'homme de préférence.
La structure tissulaire peut, comme expliqué précédemment, se présenter sous forme de bâtonnet, feuillet, patch, stick tissulaire ou sous toute autre forme d'intérêt.
Une telle structure tissulaire est aussi avantageusement utilisable en recherche *in vivo, ex vivo* ou *in vitro.*

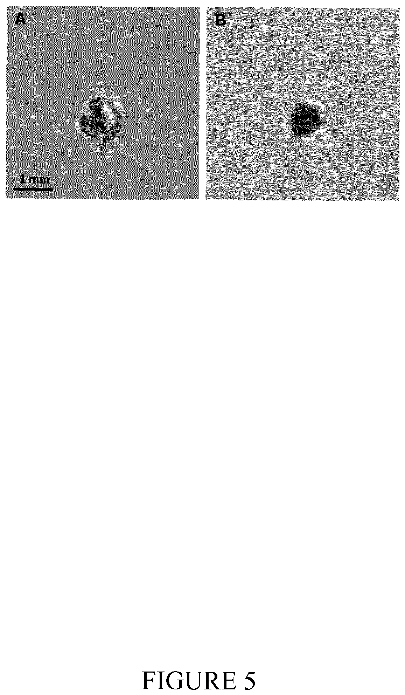

La présente description concerne aussi une méthode de suivi du développement *in vivo* d'une telle structure tissulaire.
Les agrégats cellulaires et structures cellulaires ou tissulaires décrits par les inventeurs comprennent, comme expliqué précédemment, des cellules magnétisées de sorte que leur visualisation et localisation est possible par imagerie par exemple à l'aide d'un appareil à IRM (figure 5).

La présente invention permet pour la première fois et de manière très avantageuse de contrôler la différenciation ainsi que le devenir des cellules greffées dans les tissus hôtes.

### EXEMPLES

### EXEMPLE 1 - Méthode d'agrégation appliquée à l'obtention de chondrocytes à partir de cellules souches mésenchymateuses humaines (CSMh) - Conditions de référence.

### Matériel et méthode

### • Formation des agrégats

Les cellules souches mésenchymateuses humaines (CSMh) utilisées proviennent d'aliquots commerciaux (Lonza). Ces cellules sont incubées pendant 30min avec une suspension de nanoparticules d'oxyde de fer (maghémite) à une concentration de 0.1mM en fer. Après une nuit de chasse dans un milieu de culture sans nanoparticules, 250 000, 500 000 ou 1 000 000 cellules sont déposées sur un substrat en verre (100µm d'épaisseur) placé au-dessus d'une aiguille aimantée. Ce procédé conduit à la formation d'agrégats cellulaires de forme sphérique. La force magnétique exercée sur les cellules de l'agrégat varie de 70 à 400 pN.

| Condition n° | Marquage [Fe]= | Epaisseur du substrat | Force magnétique appliquée aux cellules | Nombre de cellules dans l'agrégat |
|---|---|---|---|---|
| 1.1 | 0.1 mM | 100 µm | 70 à 400 pN | 0.25*10⁶ |
| 1.2 | 0.1 mM | 100 µm | 70 à 400 pN | 0.50*10⁶ |
| 1.3 | 0.1 mM | 100 µm | 70 à 400 pN | 1.00*10⁶ |

### • Différenciation cellulaire

Dans le cas de l'ingénierie de tissu cartilagineux, les agrégats de cellules souches mésenchymateuses sont avantageusement cultivés dans un incubateur (37°C, 5% CO₂) pendant 28 jours dans un milieu d'induction chondrogénique contenant notamment le facteur de croissance TGFβ3. Ce milieu est renouvelé deux fois par semaine.

### • Analyse du tissu formé

Au terme des 28 jours de culture dans un milieu chondrogénique, les agrégats ont été analysés en histologie et en PCR quantitative afin de mettre en évidence la présence de matrice cartilagineuse. Cette matrice se compose de fibres de collagène de type II et de protéoglycanes, qui associent un coeur protéique à des chaines de glycosaminoglycanes. Un exemple de protéoglycane trouvé en abondance dans le cartilage est l'aggrégane. La PCR quantitative permet d'évaluer l'expression des gènes des deux composants majeurs de la matrice, le collagène II et l'aggrégane. Par ailleurs, le rapport des niveaux d'expression des gènes codant pour le collagène II et le collagène I donne une indication de la progression dans la voie de différenciation chondrogénique : les cellules mésenchymateuses, non différenciées, synthétisent majoritairement du collagène I tandis que les chondrocytes produisent du collagène II en abondance.

Les colorations histologiques au bleu de toluidine et à la safranine O ont pour rôle de révéler la présence de glycosaminoglycanes par un métachromatisme du bleu vers le violet ou une coloration en orange respectivement.

### Histologie

Les échantillons sont tout d'abord fixés dans la formaline (12h d'incubation à 4°C) avant d'être congelés dans une matrice d'OCT (Optimal Cutting Temperature compound) dans un bain d'isopentane refroidi à l'azote liquide. Puis on réalise à l'aide d'un cryostat microtome des coupes de 6µm qui sont ensuite déposées sur des lames pré-traitées. Avant la coloration proprement dite, les coupes sont à nouveau fixées dans la formaline 1% pendant 10 minutes à température ambiante, et ensuite rincées dans un bain d'eau du robinet.

### Coloration au bleu de toluidine

- Marquage des lames pendant 1 à 2 minutes par une solution de bleu de toluidine à 0,5% (500mg dans 20mL d'éthanol à 95% et 80mL d'eau distillée).
- Rinçage dans un bain d'eau du robinet.
- Déshydratation : trois bains successifs dans l'éthanol 100% (2 min ; 2 min ; 5 min) et trois bains successifs dans le toluène (2 min ; 2 min ; 5 min).
- Montage des lames avec l'Eukit.

### Coloration à la safranine-O

- *Coloration à l'hématoxyline*
   ∘ Marquage pendant 3 minutes par une solution 0, 2× diluée dans l'eau distillée
   ∘ Rinçage pendant 2 minutes dans un bain d'eau du robinet
   ∘ Différenciation dans un bain d'alcool acide (1% d'acide chlorhydrique HCl dans éthanol 70%) pendant 15 secondes.
- *Coloration au Fast Green*
   ∘ Marquage pendant 3 minutes par une solution aqueuse 1 :5000 (40mg dans 200 mL d'eau).
   ∘ Rinçage rapide dans un bain d'acide acétique 1%.
- Coloration à la safranine-O
   ∘ Marquage pendant 3 minutes maximum dans une solution aqueuse de safranine-O 0,1%
   ∘ Rinçage dans un bain d'éthanol 95%.
- Déshydratation : trois bains successifs dans l'éthanol 100% (2 min ; 2 min ; 5 min) et trois bains successifs dans le toluène (2 min ; 2 min ; 5 min)
- Montage des lames avec l'Eukit.

### PCR quantitative (Polymerase Chain Reaction)

Les ARNs totaux contenus dans les cellules après la phase de différenciation sont extraits avec le kit Machery-Nagel selon le protocole indiqué par le fournisseur. On traite les ARNs obtenus avec l'ADNase afin d'éviter toute contamination par de l'ADN génomique. L'ADN complémentaire (ADNc) est ensuite synthétisé en utilisant la SuperScript II Reverse Transcriptase (Invitrogen) avec une concentration finale de 1 µg d'ARNs totaux pour 100 µL. La PCR quantitative en temps réel est effectuée avec l'appareil ABIPRISM 7900 sequence Detection System and SYBR Green dye (Applied-Biosystems), en se référant au protocole indiqué par le fournisseur. Les amorces de PCR sont construites avec le programme Primer Express (Applied Biosystems). Les niveaux de transcription d'ARNm sont normalisés par rapport au niveau d'expression d'un gène référence : RPLP0 (phosphoprotéine ribosomale acide P0). La spécificité du transcript est confirmée par une courbe de fusion effectuée à la fin de la PCR. Le seuil de détection de fluorescence établi permet de détecter un cycle seuil (Ct) et de quantifier l'expression relative d'un gène spécifique. Le niveau d'ARNm correspondant au gène d'intérêt (noté R) est exprimé par rapport au niveau du gène de référence RPLP0 : ΔCt=Ct_{R}-Ct_{RPLP0}. Pour chaque gène R, on se réfère à un échantillon témoin négatif ayant été cultivé sans milieu de différenciation. La quantité d'ARNm correspondant au gène R est exprimée relativement à ce témoin négatif, et est égal à 2^{(-ΔΔCt)}, où ΔΔCt = ΔCt_{échantillon}-ΔCt_{témoin négatif}.

Séquence des amorces utilisées : La chondrogenèse des CSMh est mesurée par le niveau d'expression de trois gènes particuliers :
∘ AGN : gène codant pour l'aggrécane, composant de la matrice cartilagineuse ;
   Fwd : TCTACCGCTGCGAGGTGAT (SEQ ID NO : 1)
   Rev : TGTAATGGAACACGATGCCTTT (SEQ ID NO :2)
∘ Col2A : gène codant pour une chaîne α du collagène II, présent dans le cartilage hyalin.
   Fwd : ACTGGATTGACCCCAACCAA (SEQ ID NO :3)
   Rev : TCCATGTTGCAGAAAACCTTCA (SEQ ID NO :4)
∘ CollA : gène codant pour une chaîne α du collagène I, présent dans le cartilage fibreux et synthétisé par des cellules mésenchymateuses non encore différenciées.
   Fwd : GCTACCCAACTTGCCTTCATG (SEQ ID NO :5)
   Rev : TTCTTGCAGTGGTAGGTGATGTTC (SEQ ID NO :6)

Pour évaluer la progression dans la différenciation, on mesure le rapport d'expression Col2A/CollA et l'expression d'AGN.

### Résultats

Les agrégats de 250 000, 500 000 ou 1 000 000 cellules souches mésenchymateuses présentent, à l'issue des 28 jours de chondrogenèse, une expression des gènes caractéristiques de la matrice cartilagineuse (AGN et Col2A/Col1A) comparable à celle du contrôle, c'est-à-dire, un agrégat de 250 000 cellules formé par centrifugation (condition standard dans la littérature).

### EXEMPLE 2 - Méthode d'agrégation appliquée à l'obtention de chondrocytes à partir de cellules souches mésenchymateuses humaines (CSMh) - Variation de la compacité des agrégats.

### Matériel et méthode

La variation de la compacité des agrégats cellulaires est obtenue en augmentant ou en diminuant la force magnétique appliquée aux cellules lors de la formation des agrégats par rapport à la condition standard présentée dans l'exemple 1. Cette force est modulée, soit par une variation du marquage magnétique des cellules, soit par un dépôt sur un substrat en verre plus épais. Les conditions expérimentales testées sont récapitulées dans le tableau suivant.

| Condition n° | Marquage [Fe]= | Epaisseur du substrat | Force magnétique appliquée aux cellules | Nombre de cellules dans l'agrégat |
|---|---|---|---|---|
| 2.1 | 0.5mM | 100µm | 140 à 900pN | 0.25×10⁶ |
| 2.2 | 0.5mM | 400µm | 140 à 700pN | 0.25×10⁶ |

Les agrégats sont formés comme expliqué dans l'exemple 1 et cultivés pendant 2 jours dans le milieu d'induction chondrogénique incluant du TGFβ3. Au cours de ces deux jours, leur dynamique de contraction est suivie.

### Résultats

Les agrégats ont la même dynamique de contraction.

### EXEMPLE 3 - Méthode d'agrégation appliquée à l'obtention de chondrocytes à partir de cellules souches mésenchymateuses humaines (CSMh) - Dépôt en géométrie « étalée ».

### Matériel et méthode

La géométrie initiale est modifiée par rapport à l'exemple 1 grâce à l'utilisation de pièces en fer doux aimantables usinées sous forme de lignes ou de croix de 0.5 µm d'épaisseur. Les conditions expérimentales utilisées sont récapitulées dans le tableau ci-dessous. Les méthodes de dépôt, d'agrégation, de culture des agrégats et d'analyse sont identiques à celles de l'exemple 1.

| Condition n° | Marquage [Fe]= | Epaisseur du substrat | Force magnétique appliquée aux cellules | Nombre de cellules dans l'agrégat | Géométrie |
|---|---|---|---|---|---|
| 3.1 | 0.05mM | 100µm | 10 à 100pN | 1.0×10⁶ | Ligne - 1cm |
| 3.2 | 0.1mM | 100µm | 20 à 240pN | 1.0×10⁶ | Ligne - 1cm |
| 3.3 | 0.1mM | 100µm | 20 à 240pN | 2.0×10⁶ | Ligne - 2cm |
| 3.4 | 0.5mM | 100µm | 50 à 450pN | 1.0×10⁶ | Ligne - 1cm |
| 3.5 | 0.5mM | 100µm | 50 à 450pN | 2.0×10⁶ | Ligne - 2cm |
| 3.6 | 2.0mM | 100µm | 100 à 1000pN | 1.0×10⁶ | Ligne - 1cm |
| 3.7 | 0.1mM | 100µm | 20 à 240pN | 4.0×10⁶ | Croix - 4×1cm |

### Résultats :

- La condition 3.6 conduit à une expression des gènes de la matrice similaire à celle du contrôle négatif (250 000 cellules confinées par centrifugation et cultivées en milieu chondrogénique sans TGFβ3).
- La condition 3.4 est plus favorable à la formation de matrice cartilagineuse que la condition 3.5.
- Les conditions 3.1, 3.2, 3.3 et 3.4 conduisent à une synthèse de matrice cartilagineuse comparable au contrôle positif (250 000 cellules confinées par centrifugation et cultivées en milieu chondrogénique avec TGFβ3).

### EXEMPLE 4 - Fusion d'agrégats de cellules souches mésenchymateuses humaines (CSMh) en cours de chondrogenèse - Application à la formation de substituts tissulaires de forme et de géométrie contrôlée.

### Matériel et méthode

Des agrégats de cellules souches mésenchymateuses humaines sont formés par condensation magnétique et cultivés en milieu chondrogénique comme expliqué dans l'exemple 1.

Le type d'aimant utilisé pour former les agrégats est soit une aiguille aimantée (comme dans l'exemple 1), soit une ligne aimantée (comme dans l'exemple 2). Plusieurs de ces agrégats (par exemple 2, 4, 7, 16) sont mis en contact à différents temps du processus de chondrogenèse (par exemple jour 0, 1, 4, 7, 10 ou 16), ce qui initie leur fusion spontanée en un unique agrégat cohésif. Cette mise en contact peut être spontanée, lorsque par exemple les agrégats sont proches les uns des autres (conditions 4.1, 4.4, 4.6, 4.7). Elle peut aussi être provoquée manuellement et maintenue grâce à la présence d'un aimant sous-jacent (aiguille ou ligne magnétique selon la condition de condensation). Des exemples de fusion provoquée sont fournis par les conditions : 4.2, 4.3, 4.5, 4.9, 4.10, 4.11, 4.12. Pour la fusion d'un grand nombre d'unités, un processus de fusion séquentiel peut aussi être utilisé. Par exemple, dans la condition 4.12 concernant la fusion de 16 unités au final, on procède tout d'abord par la fusion de 2 unités à J7 (formation de 8 doublets). Les doublets formés sont ensuite fusionnés en quadruplets à J11 (formation de 4 quadruplets). Enfin, les quatre quadruplets sont fusionnés en un feuillet à J16. Ce feuillet est le produit de la fusion séquentielle des 16 unités initiales.

Les conditions expérimentales testées sont résumées dans le tableau ci-dessous. La date de fusion désigne le nombre de jours écoulés à l'instant de fusion après l'initiation de la chondrogenèse.

| Condition n° | Condition de condensation magnétique | Nombre de cellules par agrégats | Nombre d'agrégats fusionnant | Date de fusion | Type de fusion |
|---|---|---|---|---|---|
| 4.1 | 1.1 | 0.25×10⁶ | 2 | Jour 1 | Spontanée Espacement de 2mm des aiguilles magnétiques. |
| 4.2 | 1.1 | 0.25×10⁶ | 2 | Jour 10 | Provoquée |
| 4.3 | 1.1 | 0.25×10⁶ | 2 | Jour 16 | Provoquée |
| 4.4 | 1.1 | 0.25×10⁶ | 7 | Jour 0 | Spontanée Espacement de 1.5mm des aiguilles magnétiques. |
| 4.5 | 1.1* | 0.20×10⁶ | 16 | Jour 10 | Provoquée |
| 4.6 | 3.2 | 1.0×10⁶ | 2 | Jour 4 | Spontanée Espacement de 1.5mm des lignes magnétiques. |
| 4.7 | 3.4 | 1.0×10⁶ | 2 | Jour 4 | Spontanée Espacement de 1.5mm des lignes magnétiques. |
| 4.9 | 1.1 | 0.25×10⁶ | 4 | Jour 7 | Provoquée |
| 4.10 | 1.1 | 0.25×10⁶ | 4 | Jour 9 | Provoquée |
| 4.11 | 1.1 | 0.25×10⁶ | 4 | Jour 15 | Provoquée |
| 4.12 | 1.1 | 0.25×10⁶ | 16 | Jour 8 | Provoquée (fusion séquentielle) |
| | | | | Jour 11 | |
| | | | | Jour 15 | |

| | | | | | |
|---|---|---|---|---|---|
| *Par rapport à la condition 1.1, le nombre de cellules de 0.25×10⁶ cellules par agrégat est remplacé par 0.20×10⁶cellules par agrégat. | | | | | |

### Résultats

- La proximité des aimants induisant la formation des agrégats cellulaires conduit à une fusion spontanée et précoce de ces agrégats.
- Dans le cas d'une fusion précoce (Jour 0, 1, 4), on observe une fusion totale des agrégats en un agrégat unique homogène.
- Dans le cas d'une fusion tardive (Jour 7, 10, 16), on observe une fusion des agrégats en un ensemble cohésif mais dont la structure est marquée par la présence d'unités distinctes.
- La date de mise en contact des agrégats peut être parfaitement contrôlée.
- Dans le cas d'une fusion tardive, la taille de l'agrégat final est similaire à la somme des tailles des unités le composant, alors que dans le cas d'une fusion précoce on observe une compaction supplémentaire de l'agrégat.
- Les conditions 4.4 et 4.12 conduisent à la formation d'un substitut tissulaire présentant une matrice cartilagineuse abondante.
- La condition 4.1, 4.2, 4.3, 4.9, 4.10 et 4.11 conduisent à la formation de substituts tissulaires présentant une matrice cartilagineuse.
- Dans toutes les conditions, un tissu continu est formé.
- Les conditions 4.9, 4.10, 4.11 conduisent à des niveaux d'expression des gènes de collagène II et d'aggrégane non significativement différents.
- La comparaison du niveau d'expression de collagène II des agrégats de 106 cellules, formés par fusion magnétique (conditions 4.9, 4.10, 4.11), avec celui des agrégats de 106 cellules également, formés par centrifugation, montre que ce niveau d'expression est significativement plus élevé pour les agrégats résultant de la fusion magnétique (3473±654) que pour ceux formés par la technique usuelle de centrifugation (880±291). (test statistique : Student's t test).

La présence d'un facteur de croissance induisant la chondrogenèse (TGFβ3, TGFβ1, BMP-2 ou autres) ou d'un cocktail de facteurs n'apparaît utile que pour une culture prolongée des agrégats (7-28 jours). Dans ce cas, il est alors souhaitable de créer *in vitro* un environnement favorable à l'ensemble du processus allant de la condensation cellulaire à la différenciation et la production de matrice cartilagineuse.

Dans le cadre d'une implantation précoce des agrégats de cellules souches mésenchymateuses, la première étape de condensation cellulaire peut être induite *in vitro* avant implantation ou injection des amorces ou directement *in vivo.* La suite du processus (différenciation, production de matrice) peut être stimulée par les facteurs sécrétés *in vivo* par les cellules natives. Une telle implantation précoce peut avoir lieu après 2 ou 3 jours de culture *in vitro,* et de manière préférée après une journée de culture.

### EXEMPLE 5 - Culture de cellules souches embryonnaires de souris et obtention d'agrégats sphériques

Des cellules souches embryonnaires de souris cultivées dans du DMEM (avec acides aminés essentiels et protéine LIF pour maintenir la pluripotence) par une incubation de 30 min avec les nanoparticules d'oxyde de fer pour des concentrations allant de [Fe]=0,1mM à [Fe]=2mM. La capture cellulaire par cellule embryonnaire varie alors de 1 pg à 4 pg de fer par cellule (voir courbe ci-dessous). 50 000 à 250 000 cellules ont été déposées sur un substrat de verre à distance de 100 µm de l'attracteur magnétique. La force magnétique appliquée aux cellules varie entre 50 et 200 pN. Pour toutes les conditions, des agrégats cohésifs de forme sphérique sont obtenus.

## Revendications

1. Procédé de préparation d'une structure tissulaire implantable *in vivo* chez l'animal, ledit procédé étant réalisé en l'absence de matrice de soutien tridimensionnelle exogène et comprenant la mise en oeuvre d'un procédé d'agrégation de cellules souches ou de cellules en voie de différenciation comprenant:
a) la mise en contact *in vitro* ou *ex vivo* de cellules souches ou de cellules en voie de différenciation avec des particules magnétiques, ladite mise en contact permettant l'obtention de cellules magnétisées sur lesquelles sont fixées ou dans lesquelles sont internalisées des particules magnétiques, et
b) l'exposition desdites cellules magnétisées à un champ magnétique, ladite exposition permettant la formation d'un agrégat cellulaire.

2. Procédé selon la revendication 1, dans lequel les cellules souches sont des cellules souches mésenchymateuses (CSM), des cellules souches embryonnaires (CSE) ou des cellules progénitrices induites.

3. Procédé selon l'une des revendications 1 ou 2, dans lequel le champ magnétique est un champ focalisé.

4. Procédé selon l'une quelconque des revendications précédentes, dans lequel l'étape b) d'exposition des cellules magnétisées à un champ magnétique consiste en l'exposition desdites cellules à une ou plusieurs formes aimantées.

5. Procédé selon la revendication 4 dans lequel les cellules magnétisées sont exposées à une forme aimantée et ladite forme est choisie parmi un point, la pointe ou l'extrémité d'une aiguille tronquée, une ligne, une croix, une étoile, un anneau, un disque, un cercle, ou plusieurs de l'une et/ou l'autre desdites formes.

6. Procédé selon l'une quelconque des revendications 1 à 5 permettant d'obtenir un agrégat cellulaire ledit procédé étant réalisé sans étape de centrifugation et de préférence en l'absence de facteur de différenciation.

7. Procédé selon l'une quelconque des revendications 1 à 6, dans lequel 0.5, 1 ou 2 millions de cellules souches ou de cellules en voie de différenciation sont utilisées.

8. Procédé selon l'une quelconque des revendications 1 à 7, **caractérisé en ce qu'**il comprend en outre une étape de culture d'un ou de plusieurs agrégats cellulaires, constitués de cellules identiques ou différentes, obtenus à l'issue dudit procédé d'agrégation.

9. Procédé selon la revendication 8, **caractérisé en ce qu'**il est réalisé en l'absence de facteur de croissance durant le premier jour ou les 2, 3, 4, 5, 6, 7 ou 8 premiers jours de l'étape de culture suivant la mise en oeuvre du procédé d'agrégation.

10. Procédé selon la revendication 8, **caractérisé en ce qu'**il permet la préparation d'une structure cartilagineuse et est mis en oeuvre en l'absence de facteur de croissance, de préférence en l'absence de TGFβ1, TGFβ3 et/ou BMP-2, durant l'étape d'agrégation au moins.

11. Procédé selon l'une quelconque des revendications 8 à 10, **caractérisé en ce que** l'étape de culture est réalisée en présence d'un champ magnétique créé à l'aide d'une ou de plusieurs sources magnétiques.

12. Agrégat cellulaire pour une utilisation pour une implantation *in vivo* chez l'animal, ledit agrégat cellulaire étant dépourvu de matrice de soutien tridimensionnelle exogène et comprenant des cellules souches magnétisées en cours de différenciation et/ou des cellules magnétisées en cours de différenciation, des particules magnétiques étant fixées sur, ou internalisées dans, lesdites cellules magnétisées.

13. Utilisation d'un ou plusieurs agrégats cellulaires dépourvu(s) de matrice de soutien tridimensionnelle exogène et comprenant des cellules souches magnétisées, des cellules souches magnétisées en cours de différenciation, et/ou des cellules magnétisées en cours de différenciation, des particules magnétiques étant fixées sur, ou internalisées dans, lesdites cellules magnétisées, en tant qu'amorces tissulaires pour obtenir, *in vitro* ou *ex vivo,* une structure tissulaire d'intérêt.

14. Substitut tissulaire dépourvu de matrice de soutien tridimensionnelle exogène comprenant (i) des cellules magnétisées différenciées sur lesquelles sont fixées ou dans lesquelles sont internalisées des particules magnétiques et (ii) une matrice extracellulaire sécrétée par lesdites cellules magnétisées différenciées.

15. Substitut tissulaire selon la revendication 14, **caractérisée en ce que** les cellules magnétisées différenciées sont des cellules cartilagineuses magnétisées et **en ce que** la matrice extracellulaire est une matrice extracellulaire cartilagineuse.

16. Substitut tissulaire selon l'une des revendications 14 ou 15, **caractérisée en ce qu'**elle se présente sous la forme d'un bâtonnet, d'un feuillet, d'un patch, ou d'un stick tissulaire.

## Patentansprüche

1. Verfahren zur Herstellung einer Gewebestruktur, die *in vivo* in Tiere implantiert werden kann, wobei das Verfahren ohne exogene dreidimensionale Trägermatrix durchgeführt wird und die Durchführung eines Verfahrens zur Aggregation von Stammzellen oder von Differenzierungszellen mit folgenden Schritten umfasst:
a) Berührung *in vitro* oder *ex vivo* von Stammzellen oder Differenzierungszellen mit magnetischen Partikeln, wobei durch die Berührung magnetisierte Zellen erzielt werden können, an denen magnetische Partikel fixiert, oder in denen sie internalisiert sind, und
b) Exposition der magnetisierten Zellen gegenüber einem Magnetfeld, wobei die Exposition die Bildung eines Zellaggregats ermöglicht.

2. Verfahren nach Anspruch 1, wobei die Stammzellen mesenchymale Stammzellen (MSC), embryonale Stammzellen (ESC) oder induzierte Vorläuferzellen sind.

3. Verfahren nach einem der Ansprüche 1 oder 2, wobei das Magnetfeld ein fokussiertes Feld ist.

4. Verfahren nach einem der vorstehenden Ansprüche, wobei Schritt b) mit Exposition der magnetisierten Zellen gegenüber einem Magnetfeld darin besteht, die Zellen einer oder mehreren magnetischen Formen auszusetzen.

5. Verfahren nach Anspruch 4, wobei die magnetisierten Zellen einer magnetisierten Form ausgesetzt sind und die Form unter einem Punkt, der Spitze oder dem Ende einer abgestumpften Nadel, einer Linie, einem Kreuz, einem Stern, einem Ring, einer Scheibe, einem Kreis oder mehreren der einen und/oder der genannten Formen ausgewählt ist.

6. Verfahren nach einem der Ansprüche 1 bis 5, wobei ein Zellaggregat erzielt werden kann, wobei das Verfahren ohne Zentrifugierungsschritt und vorzugsweise ohne Differenzierungsfaktor durchgeführt wird.

7. Verfahren nach einem der Ansprüche 1 bis 6, wobei 0,5, 1 oder 2 Millionen Stammzellen oder Differenzierungszellen verwendet werden.

8. Verfahren nach einem der Ansprüche 1 bis 7, **dadurch gekennzeichnet, dass** es ferner einen Schritt mit Kultivierung eines oder mehrerer zellulärer Aggregate umfasst, die aus identischen oder unterschiedlichen Zellen bestehen und als Ergebnis des Aggregationsprozesses erzielt wurden.

9. Verfahren nach Anspruch 8, **dadurch gekennzeichnet, dass** es ohne Wachstumsfaktor am ersten Tag oder an den ersten 2, 3, 4, 5, 6, 7 oder 8 Tagen des Kultivierungsschritts nach dem Aggregationsprozess durchgeführt wird.

10. Verfahren nach Anspruch 8, **dadurch gekennzeichnet, dass** es die Herstellung einer Knorpelstruktur ermöglicht und ohne Wachstumsfaktor, vorzugsweise ohne TGFβ1, TGFβ3 und/oder BMP-2, zumindest während des Aggregationsschritts, durchgeführt wird.

11. Verfahren nach einem der Ansprüche 8 bis 10, **dadurch gekennzeichnet, dass** der Kultivierungsschritt bei vorhandenem Magnetfeld durchgeführt wird, das durch eine oder mehrere Magnetquellen erzeugt wird.

12. Zellaggregat zur Verwendung für eine *in vivo-*Implantation beim Tier, wobei das Zellaggregat ohne exogene dreidimensionale Trägermatrix ist und magnetisierte Stammzellen im Differenzierungsprozess und/oder magnetisierte Zellen im Differenzierungsprozess umfasst, wobei magnetische Partikel an den magnetisierten Zellen fixiert oder darin internalisiert sind.

13. Verwendung eines oder mehrerer Zellaggregate ohne exogene dreidimensionale Trägermatrix(en), die magnetisierte Stammzellen, magnetisierte Stammzellen im Differenzierungsprozess und/oder magnetisierte Zellen im Differenzierungsprozess umfasst(en), wobei magnetische Partikel als Gewebeprimer an den magnetisierten Zellen fixiert oder in diesen internalisiert sind, um *in vitro* oder ex *vivo* eine Gewebekonstruktion von Interesse zu erzielen.

14. Gewebeersatz ohne exogene dreidimensionale Trägermatrix umfassend (i) differenzierte magnetisierte Zellen, an denen magnetische Partikel fixiert oder darin internalisiert sind, und (ii) eine extrazelluläre Matrix, die von den differenzierten magnetisierten Zellen abgesondert wird.

15. Gewebeersatz nach Anspruch 14, **dadurch gekennzeichnet, dass** die differenzierten magnetisierten Zellen magnetisierte Knorpelzellen sind, und dass die extrazelluläre Matrix eine extrazelluläre Knorpelmatrix ist.

16. Gewebeersatz nach einem der Ansprüche 14 oder 15, **dadurch gekennzeichnet, dass** er in Form eines Gewebestäbchens, eines -blättchens, eines -Patch oder eines -sticks vorliegt.

## Claims

1. Process for preparing a tissue structure which is implantable *in vivo* in animals, said process being carried out in the absence of exogenous three-dimensional support matrix and comprising the implementation of a process for aggregation of stem cells or of cells undergoing differentiation comprising:
(a) bringing stem cells or cells undergoing differentiation into contact, *in vitro* or ex *vivo,* with magnetic particles, said bringing into contact making it possible to obtain magnetised cells to which are bound, or in which are internalised, magnetic particles, and
(b) exposing said magnetised cells to a magnetic field, said exposure enabling the formation of a cell aggregate.

2. Process according to claim 1, wherein the stem cells are mesenchymal stem cells (MSCs), embryonic stem cells (ESCs) or induced progenitor cells.

3. Process according to one of claims 1 or 2, wherein the magnetic field is a focused field.

4. Process according to any one of the preceding claims, wherein step b) of exposing the magnetised cells to a magnetic field consists of exposure of said cells to one or more magnetic shapes.

5. Process according to claim 4, wherein the magnetised cells are exposed to a magnetic shape and said shape is chosen from a spot, the tip or the end of a truncated needle, a line, a cross, a star, a ring, a disc, a circle, or several of one and/or the other of said shapes.

6. Process according to any one of claims 1 to 5, for obtaining a cell aggregate, said process being carried out without a centrifugation step and preferably in the absence of differentiation factor.

7. Process according to any one of claims 1 to 6, wherein 0.5, 1 or 2 million stem cells or cells undergoing differentiation are used.

8. Process according to any one of claims 1 to 7, **characterized in that** it further comprises a step of culturing one or more cell aggregates, consisting of identical or different cells, obtained at the end of said aggregation process.

9. Process according to claim 8, **characterized in that** it is carried out in the absence of growth factor during the first day or the first 2, 3, 4, 5, 6, 7 or 8 days of the culturing step following the implementation of the aggregation process.

10. Process according to claim 8, **characterized in that** it enables the preparation of a cartilaginous structure and is implemented in the absence of growth factor, preferably in the absence of TGFβ1, TGFβ3 and/or BMP-2, at least during the aggregation step.

11. Process according to any one of claims 8 to 10, **characterized in that** the culturing step is carried out in the presence of a magnetic field created using one or more magnetic sources.

12. Cell aggregate for use for *in vivo* implantation in animals, said cell aggregate being devoid of exogenous three-dimensional support matrix and comprising magnetised stem cells undergoing differentiation and/or magnetised cells undergoing differentiation, magnetic particles being bound to, or internalised in, said magnetised cells.

13. Use of one or more cell aggregates devoid of exogenous three-dimensional support matrix and comprising magnetised stem cells, magnetised stem cells undergoing differentiation, and/or magnetised cells undergoing differentiation, magnetic particles being bound to, or internalised in, said magnetised cells, as tissue initiators for obtaining, *in vitro* or ex *vivo,* a tissue structure of interest.

14. Tissue substitute devoid of exogenous three-dimensional support matrix comprising (i) differentiated magnetised cells to which are bound, or in which are internalised, magnetic particles and (ii) an extracellular matrix secreted by said differentiated magnetised cells.

15. Tissue substitute according to claim 14, **characterized in that** the differentiated magnetised cells are magnetised cartilaginous cells and **in that** the extracellular matrix is a cartilaginous extracellular matrix.

16. Tissue substitute according to one of claims 14 or 15, **characterized in that** it is in the form of a tissue stick, patch, sheet or rod.


### SEQUENCE LISTING

<110> Centre National de la Recherche Scientifique
<120> PROCEDES D'AGGREGATION ET DE DIFFERENCIATION DE CELLULES SOUCHES MAGNETISEES
<130> B1224PC00
<160> 6
<170> PatentIn version 3.5
<210> 1
   <211> 19
   <212> DNA
   <213> artificial sequence
<220>
   <223> amorce Fwd_ AGN
<400> 1
   tctaccgctg cgaggtgat 19
<210> 2
   <211> 22
   <212> DNA
   <213> artificial sequence
<220>
   <223> amorce Rev_AGN
<400> 2
   tgtaatggaa cacgatgcct tt 22
<210> 3
   <211> 20
   <212> DNA
   <213> artificial sequence
<220>
   <223> amorce Fwd_Col2A
<400> 3
   actggattga ccccaaccaa 20
<210> 4
   <211> 22
   <212> DNA
   <213> artificial sequence
<220>
   <223> amorce Rev_Col2A
<400> 4
   tccatgttgc agaaaacctt ca 22
<210> 5
   <211> 21
   <212> DNA
   <213> artificial sequence
<220>
   <223> amorce Fwd_Col1A
<400> 5
   gctacccaac ttgccttcat g 21
<210> 6
   <211> 24
   <212> DNA
   <213> artificial sequence
<220>
   <223> amorce Rev_Col1A
<400> 6
   ttcttgcagt ggtaggtgat gttc 24